# EUROPÄISCHE PATENTSCHRIFT

(11) **EP 0 368 103 B1**
(45) Veröffentlichungstag und Bekanntmachung des Hinweises auf die Patenterteilung: **23.02.1994**
(21) Anmeldenummer: 89120044.6
(22) Anmeldetag: 28.10.1989
(51) Int. Cl.: B65D 39/18, C08J 7/04

(54) **Verfahren zum Abfüllen und Verschliessen von Behältern mit pharmazeutisch reinem Inhalt**
Method of filling and closing of containers with pharmaceutically pure contents
Procédé de remplissage et de fermeture de récipients de contenu pharmaceutique pur

(30) Priorität: 07.11.1988 US 267828
(43) Veröffentlichungstag der Anmeldung: 16.05.1990
(73) Patentinhaber: Pharma-Gummi Wimmer West GmbH, 52249 Eschweiler (DE)
(72) Erfinder: Romberg, Val G., Upper Darby Pennsylvania 19082 (US); Wayne, Curry T., Pottstown Pennsylvania 19464 (US); Kiang, Patty H., Collegeville Pennsylvania 19426 (US)
(74) Vertreter: Schmitt, Hans, Dipl.-Ing.

(56) Entgegenhaltungen:
- WO-A-88/08012

## Beschreibung

Die Erfindung betrifft ein verfahren gemäß dem Oberbegriff von Anspruch 1.

Hintergrund der Erfindung : Für Plastik- und insbesondere Glasflaschen, die pharmazeutische Produkte zum Inhalt haben, kennt man seit vielen Jahren als bewährtes Verschlußsystem Stopfen aus elastomerem Werkstoff. Insbesondere hat sich die Kombination von Glasbehältern, die mit einem Gummistopfen verschlossen sind, in weiten Anwendungsbereichen für die Verpackung von pharmazeutischen Wirkstoffen bewährt. Dabei wird sowohl ein sicherer Verschluß des pharmazeutischen Wirkstoffes (Medizin) als auch ein leichter Zugriff ermöglicht, weil man durch das elastomere Verschlußteil, z. B. durch einen Gummistopfen, leicht eine Hohlnadel hindurchführen kann, um eine gewünschte Menge eines z. B. flüssigen pharmazeutischen Wirkstoffes zu entnehmen, ohne daß der Behälterinhalt anderweitig berührt wird. Auch wenn der pharmazeutische Wirkstoff in der Plastik- oder Glasflasche pulverförmig ist, ermöglicht die vorerwähnte Behälter-Stopfen-Kombination eine leichte Handhabung : Man kann das elastomere Verschlußteil mit einer Hohlnadel durchstechen, um den pulverförmigen pharmazeutischen Wirkstoff durch Hinzufügen von Flüssigkeit zu aktivieren. Eine solche Flüssigkeit kann z. B. chemisch reines oder destilliertes Wasser sein. Der so aktivierte chemische Wirkstoff kann dann in der vorbeschriebenen Weise entnommen werden. Sowohl dabei als auch beim Aktivieren verbleibt der pharmazeutische Wirkstoff in einem mittels des elastomeren Verschlußteiles gut abgeschlossenen Behälter geschützt.

Die Flaschen werden in aller Regel in verhältnismäßig großen Stückzahlen pro Zeiteinheit in Abfüll- und Verschließanlagen mit dem pharmazeutischen Wirkstoff gefüllt und durch elastomere Verschlußteile, z. B. durch Gummistopfen, verschlossen. Häufig werden diese Stopfen od. dgl. Verschlußteile auch z. B. durch Bördelkappen od. dgl. Haltemittel an gläsernen Medikamentenflaschen od. dgl. Behälter festgelegt. In bekannten Abfüll- und Verschließanlagen werden Gummistopfen od. dgl. elastomere Verschlußteile zunächst in einem Magazin aufbewahrt und dann zu einer Verschlußvorrichtung transportiert. Dies erfolgt üblicherweise mit Hilfe von Zentrifugalkraft- oder Vibrationsförderern oder auch unter Zuhilfenahme der Schwerkraft. Für ein ökonomisches und störungsfreies Arbeiten solcher Abfüll- und Verschließanlagen ist u. a. wesentlich, daß die Gummistopfen od. dgl. elastomere Verschlußteile nicht untereinander, z. B. im Magazin, aneinanderhängen oder an Teilen der Stopfen-Transporteinrichtung hängenbleiben oder dort nicht gebremst werden und damit ihre Transportgeschwindigkeit verlangsamen. Auch ist wesentlich, daß diese Gummistopfen od. dgl. leicht, z. B. in Bördelkappen od. dgl. einzubringen sind.

Die Abfüll- und Verschließanlagen dienen nämlich in aller Regel sowohl zum Füllen der Medikamentenflasche als auch zum Einbringen des Gummistopfens od. dgl. in den Hals der Medikamentenflasche. In aller Regel werden die Stopfen od. dgl. auf sogenannten Linien gleitend der Stelle zugeführt, an der sie in der Abfüll- und Verschließanlage in den Hals der Medikamentenflasche eingebracht werden. Die zum Befördern der Stopfen dienende Zufuhrvorrichtung weist gewöhnlich schienenartige Gleiteinrichtungen für diese Stopfen auf, die normalerweise aus rostfreiem Stahl oder anderem Werkstoff bestehen, das für pharmazeutische Verwendungszwecke extrem gut saubergehalten werden kann. Dabei ergeben sich einige Probleme bezüglich einer möglichst vorteilhaften Ausbildung der Gummistopfen od. dgl. elastomere Verschlußteile. Deren Fähigkeit, leicht auf einer entsprechenden schienenartigen Gleiteinrichtung der Zufuhrvorrichtung von Abfüll- und Verschließanlagen entlangzugleiten, ist direkt vom Reibungskoeffizienten des Stopfens od. dgl. abhängig. Dabei sind niedrige Beträge des Reibungskoeffizienten wünschenswert. Auch ist wesentlich, daß die Gummistopfen od. dgl. Verschlußteile während ihres Transportes innerhalb der Abfüll- und Verschließanlage nicht aneinangerkleben bzw. aneinanderkleben bleiben.

Höhere Reibungskoeffizienten bei gebräuchlichen Gummistopfen od. dgl., die z. B. in einer Abfüll- und Verschließanlage oder in einer anderen pharmazeutischen Vorrichtung zu befördern waren, stellten oft den Begrenzungsfaktor für die Arbeitsgeschwindgkeit solcher Abfüll- und Verschlußanlagen od. dgl. Vorrientungen dar. Typischerweise haben Gummistopfen od. dgl. elastomere Verschlußteile der eingangs erwähnten Art , wie sie für pharmazeutische Verschlüsse verwendet werden, Reibungskoeffzierten von wenigstens 1,6 bis 1,9. Dieser vergleichsweise hohe Reibungskoeffizient stellt ein erhebliches Hindernis für eine schnelle Beförderung solcher Gummistopfen od. dgl. dar.

Um die Handhabungsmöglichkeit von Gummistopfen od. dgl. und ihre Verarbeitbarkeit namentlich im vorbeschriebenen pharmazeutischen Bereich zu verbessern, hat man auch bereits diese Gummistopfen od. dgl. elastomere Verschlußteile oberflächlich silikonisiert. Dies hat aber bekanntermaßen auch Nachteile. Durch Beschichtung der Außenseite des Gummistopfens od. dgl. mit Silikonöl erreicht man zwar einerseits eine Verbesserung der Gleitfähigkeit dieser Gummistopfen od. dgl. insbesondere in der Zuführvorrichtung; andererseits erhöht eine solche Verwendung von Silikonöl die Anzahl der Partikel, die bei entsprecherden Untersuchungen von verschiedenen Medikamentenlösungen in diesen aufgefunden wurde. Außerdem lagert sich im Verlaufe der Verarbeitung vieler Stopfen mehr und mehr von dem Silikonöl auf den Fördereinrichtungen der Abfüll- und Verschließmaschine ab, was zu unerwünschten und unkontrollierbaren Kontaminationen der Stopfen führt und einen erhöhten Reinigungsaufwand für die Anlagen mit sich bringt. Silikonöl ist ein unerwünschter Verunreiniger von Arzneimitteln und beeinträchtigt die allgemeine Akzeptanz damit behandelter Gummistopfen od. dgl. Die einsetzbare Menge an Silikonöl ist darüberhinaus so gering, daß die einzelnen Stopfen zwar daran gehindert werden aneinanderzukleben, jedoch der Reibungskoeffizient der Gummistopfen od. dgl. nicht so verbessert wird, daß insbesondere in Hochleistungsverschließmaschinen eine gleichmäßig schnellere Förderung erreicht werden kann.

Bei Gummistopfen od. dgl. elastomeren Verschlußteile für Medikamantenflaschen od. dgl. Behälter mit pharmazeutischem, reinem Inhalt darin besteht gleichzeitig noch die Bedingung, daß der Stopfenwerkstoff so inert wie möglich sein soll, wenn er mit dem pharmazeutischen Produkt wie z. B. einem Arzneimittel im Kontakt kommt. Dabei kommt hinzu, daß bei der Herstellung von elastomeren Verschlußteilen in vielen Fällen bestimmte Spuren-Metalle verwerdet werden und es ist erwünscht, daß diese herstellungsbedingten Spuren-Metalle nicht in einem praktisch ins Gewicht fallenden Umfang durch das Arzneimittel, andere pharmazeutische Flüssigkeiten od. dgl. Flascheninhalt, die in Kontakt mit dem elastomeren Verschlußteil kommen, extrahiert bzw. herausgewaschen werden. Insbesondere bezieht sich dies auf Metalle wie Kalzium, Aluminium und Schwermetalle, z. B. Zink und Blei.

Um die vorbeschriebenen Nachteile von vorbekannten Gummistopfen od. dgl. Verschlußteile für Medikamentenflaschen wenigstens teilweise zu beseitigen, hat man auch bereits ein elastomeres Verschlußteil für einer Behälter mit pharmazeutischem, reiner Inhalt darin geschaffen, das sich dadurch kennzeichnet, daß das Verschlußteil od. dgl. ein elastomeres Grundteil (Basisteil) und eine vollständige Polyparaxylylen-Beschichtung auf diesem Basisteil aufweist, wobei diese Beschichtung eine Dicke vor 0,5 Mikrometer bis 2,0 Mikrometer aufweist (WO 88/08 012). Die Polyparaxylylen-Beschichtung dieses vorbekannten Gummistopfens bewirkt einen merkbar verkleinerten Reibungskoeffizienten, der eine schnelle und störungsfreie Verarbeitung dieser Stopfen in einer Abfüll- und Verschließanlage begünstigt. Dabei lassen sich derartige Verschlußteile mit einem um so niedrigeren Reibungskoeffizienten schaffen, je dicker die Beschichtung mit Poly-(Paraxylylen) ist.

Derartig in vorbekannter Weise verschlossene Medikamentenflaschen oder dergleichen Behälter jedoch, deren Inhalt unter Stickstoff abgefüllt ist, tauschen diesen Stickstoff in Stunden gegen Luft aus. Herrscht in entsprechenden gläsernen Medikamentenflaschen ein Vakuum, verlieren solche Glasflaschen, die mit den vorbekannten elastomeren Verschlußteilen verschlossen sind, beim Stehenlassen schon nach kurzer Zeit das Vakuum vollständig oder zu einem erheblichen Teil.

Es besteht daher die Aufgabe, ein Verfahren Zu schaffen, welches ein Abfüllen und Verschließen von Behältern mit pharmazeutisch reinem Inhalt vor allem in den spezifischen Anwendungsfällen erlaubt, in denen die Verhinderung eines Gasaustausches oder eine verbesserte Vakuumdichtigkeit gefordert ist, ohne daß damit gleichzeitig der Abfüll- und Verschließvorgang durch einen höheren Reibungskoeffizienten wesentlich beeinträchtigt wäre. Dabei sollen insbesondere solche Ausführungsformen von Verschlußstopfen in Relation zueinander gestellt werden, deren Anwendungsgebiete besondere Erfordernisse stellen, so daß man in die Lage versetzt wird, zu bestimmten, vom pharmazeutischen Wirkstoff, seiner gewünschten Umgebung sowie der Verpackungsart her vorgegebene Verhältnisse durch entsprechende Ausbildung des elastomeren Verschlußteiles erfüllen zu können. Dabei ist regelmäßig vom Verschlußstopfen oder dergleichen elastomeren Verschlußteil die Bedingung eines verkleinerten Reibungskoeffizienten zu erfüllen, wie er für eine vergleichsweise schnelle und störungsfreie Verarbeitung in einer Abfüll- und Verschließanlage gewünscht wird.

Die erfindungsgemäße Lösung dieser Aufgabe besteht in den kennzeichnenden Merkmalen von Anspruch 1.

Es gehört mit zur Erfindung, daß mittels Versuche festgestellt wurde, daß ein Reibungskoeffiziert unter 1,0 ausreicht, um einer Gummistopfen od. dgl. elastomeres Verschlußteil auf den Sortier-, Fördereinrichtungen od. dgl. einer Abfüllanlage gut und ohne zusätzliche Behandlung dieses elastomeren Verschlußteils zu verarbeiten. Es wurde festgestellt, daß auch noch für eine Beschichtungsdicke vor unterhalb von 0,5 bis 0,1 Mikrometer eine ausreichende Verminderung des Reibungskoeffizienten erreicht wird. Wie nachstehend in Tabellen näher aufgezeigt, haben erfindungsgemäß durchgeführte Versuche belegt, daß bis zu einer Poly-(paraxylylen)-Schichtdicke von 0,5 Mikrometer der Reibungskoeffizient immer noch unter 1,0 bleibt. Es gehört ebenfalls mit zur Erfindung, daß festgestellt werden konnte, daß bei einer Schichtdicke von 0,1 bis 0,5 Mikrometer bei Poly-(paraxylylen) eine wesentlich bessere Gas-Abdichtung erreicht wird als bei den vorbekannten elastomeren Verschlußteilen mit einer durchgehenden Poly-(paraxylylen)-Beschichtung von einer Dicke von 0,5 bis 2,0 Mikrometer.

Das erfindungsgemäße Verfahren ermöglicht also nicht nur - wie beim Abfüllen und Verschließen mit den in der PCT-Offenlegungsschrift PCT/DE 88 00 224 (= Internationale Veröffentlichungsnummer: WO 88/08 012) beschriebenen Verschlußstopfen -, daß über längere Zeit der in der Medikamentenflasche enthaltene pharmazeutische Wirkstoff gegen ein Eindringen von Feuchtigkeit und Keimen geschützt ist, sondern es wird insbesondere die Gasdichtigkeit durch die besondere Ausbildung des elastomeren Verschlußteils verbessert. Entsprechend den Anforderungen des jeweiligen pharmazeutischen Wirkstoffes und seiner in der Medikamentenflasche vorgesehenen Umgebung kann u. U. eine gewisse Verminderung der Sperrwirkung der Poly-(paraxylyler)-Beschichtung gegen die Extraktion von Metallionen in Kauf genommen werden. Man erhält ein elastisches Verschlußteil, das in entsprechenden, spezifischen Anforderungen insbesondere der Verhinderung eines Gasaustausches und der Vakuumdichtigkeit verbessert ist.

Eine Weiterbildung der Erfindung sieht vor, daß die Beschichtungsdicke (d) der Poly-(paraxylylen)-Beschichtung derart gewählt wird, daß der Reibungskoeffizient des so beschichteten Verschlußteiles kleiner als 1 ist. Dies begünstigt insbesondere eine gute und schnelle Verarbeitbarkeit auf einer Abfüll- und Verschließanlage. Weitere Verbesserungen des Reibungskoeffizienten erfolgen -wie Versuche gezeigt haben- dadurch, daß man im Bereich der Dicke der Beschichtung aus Poly-(paraxylylen) diese Beschichtungsdicke größer wählt.

Zweckmäßig ist es, wenn die Beschichtungsdicke der Poly-(paraxylylen)-Beschichtung derart gewählt wird, daß die Vakuumretension des Verschlußteiles während 24 h eine Erhöhung eines Vakuums von ursprünglich -846 mbar auf einen Wert größer als -300 mbar nicht zuläßt.

Die Verminderung des Reibungskoeffizienten einerseits und die Vakuumretension andererseits verlangen also bezüglich der Dicke der Beschichtung aus Poly-(paraxylylen) unterschiedliche Maßnahmen. Die Erfindung zeigt hier die Maßnahmen auf, welche jeweils dann zu treffen sind, wenn die eine oder die andere Forderung (Verminderung des Reibungskoeffizienten oder Verminderung der Vakuumretension) die Priorität erhält.

Vorteilhaft ist es, wenn die Beschichtungsdicke (d) der Poly-(paraxylylen)-Beschichtung derart gewählt wird, daß der Gewichtsverlust einer Flasche, die zur Hälfte mit einer Lösung von 0,9 % Kochsalz und 0,4 % Phenol gefüllt ist, bei 16-stündiger Lagerung im Hochvakuum von -1016 mbar nicht größer ist als 0,9 mg. Eine Verringerung des vorerwähnten Gewichtsverlustes begünstigt man dadurch, daß man die Dicke der Poly-(paraxylylen)-Beschichtung vermindert.

Vorteilhaft ist es, wenn die Beschichtungsdicke der Poly-(paraxylylen)-Beschichtung derart gewählt wird, daß der Austausch von Stickstoff gegen Luft nach 24 Stunden 2% der ursprünglich in der Medikamentenflasche vorhandenen Stickstoffmenge nicht überschreitet. Auch hier haben Versuche gezeigt, daß durch die Verminderung der Dicke der Poly-(paraxylylen)-Beschichtung die Austauschrate von Stickstoff gegen Luft vermindert wird.

Schließlich besteht eine besondere Ausführungsweise des erfindungsgemäßen Verfahrens darin, daß die Dicke der Poly-(paraxylylen)-Beschichtung aerart gewählt wird, daß die zum Aufsetzen auf die Medikamentenflaschen benötigte Eindrückkraft etwa 75% bis etwa 50% unter der eines vergleichbaren, jedoch unbeschichteten Verschlußteiles liegt. Um die Eindrückkraft deutlich gegenüber unbeschichteten, vergleichbaren elastomeren Verschlußteilen zu vermindern, wählt man eine vergleichsweise große Beschichtungsdicke innerhalb des Bereiches von 0,1 Mikrometer bis 0,5 Mikrometer.

### 1. Eindrückkraft

Die Eindrückkraft von Infusionsstopfen nach DIN 58 363 wurde in Abhängigkeit der Schichtdicke der Poly-(paraxylylen)-Beschichtung gemessen :

| Schichtdicke [Mikrometer] | Eindrückkraft [N] |
|---|---|
| 1.0 | 75 |
| 0.8 | 75 |
| 0.6 | 90 |
| 0.4 | 110 |
| 0.2 | 135 |
| 0.1 | 145 |
| silikonisiert | 185 |
| unsilikonisiert | 200 |

### 2. Reibungskoeffizient

(Der Reibungskoeffizient ist der Tangens des Winkels einer schiefen Ebene, bei dem die elastischen Verschlüsse bei Belastung mit einem Gewicht zu gleiten beginnen :

| Schichtdicke | COF (Reibungskoeffizient) |
|---|---|
| 1.0 | 0.36 |
| 0.8 | 0.38 |
| 0.6 | 0.47 |
| 0.4 | 0.55 |
| 0.2 | 0.74 |
| 0.1 | 0.93 |
| silikonisiert | 1.73 |
| unsilikonisiert | 1.88 |

### 3. Gasaustausch

### a) Verlust von Stickstoff

Die Vials (Medikamentenflaschen) wurden mit N₂ unter 1 bar gefüllt und nach 24 h der Verlust an N₂ gemessen. Gemessen wurden verschiedene Stopfenausführungen :

| Beschichtungsdicke [Mikrometer] | Verlust an N₂ (%) Stopfenausführung | | |
|---|---|---|---|
| | 1 | 2 | 3 |
| 0.5 | 3.8 | 1.5 | 1.6 |
| 0.4 | 1.9 | 1.1 | 1.1 |
| 0.3 | 0.05 | 0.95 | 0.95 |
| 0.2 | 0.4 | 1.0 | 0 |
| 0.1 | 0 | 0.55 | 0 |
| 0 | 0 | 0.1 | 0 |

### b. Vakuumverlust

Anfangsvakuum = 846 mbar;

| Beschichtungsdicke [Mikrometer] | Druck (mbar) nach Stunden : | | | |
|---|---|---|---|---|
| | 1 | 2 | 4 | 24 |
| 1 | -779 | -694 | -508 | -169 |
| 0.5 | -812 | -779 | -745 | -339 |
| 0.25 | -846 | -846 | -813 | -830 |
| 0 | -846 | -846 | -846 | -846 |

### c. Gewichtsverlust :

Gemessen wird der Gewichtsverlust einer zur Hälfte mit einer Lösung von 0,9% Kochsalz und 0,4% Phenol in Wasser nach 16stündiger Lagerung bei Raumtemperatur und Hochvakuum von 1016 mbar. Diese Lösung entspricht einem in der pharmazeutischen Industrie häufig vorkommenden Lösungsmittel für Medikamente und ist aufgrund ihrer Oberflächenspannung besonders für solche Versuche geeignet.

| Beschichtungsdicke [Mikrometer] | Gewichtsverlust [mg] |
|---|---|
| 0 | 0.29 |
| 0.1 | 0.07 |
| 0.2 | 0.47 |
| 0.3 | 0.43 |
| 0.4 | 0.87 |

Die vorstehenden Versuche zeigen zunächst auf, daß man überraschenderweise im Bereich der Beschichtungsdicke aus Poly-(paraxylylen) von 0,1 bis 0,5 Mikrometer bei elastomeren Verschlußteilen ein deutlich unterschiedliches Abdichtverhalten erreichen und dabei einen Reibungskoeffizienten des elastischen Verschlußteils erhalten kann, der eine zusätzliche Silikonisierung zur Verarbeitung auf einer Hochleistungs-Abfüll- und Verschließanlage ohne Schwierigkeiten zuläßt. Zusätzlich werden in Verbindung mit den Versuchen und insbesondere den Merkmalen in der Unteransprüchen Gestaltungsmöglichkeiten aufgezeigt, die das Setzen von unterschiedlichen Prioritäten (Vakuum-Verlust, Stickstoff-Verlust, Veränderung der Eindrückkraft und Reibungskoeffizient) beim elastomeren Verschlußteil bzw. der Behälter-Verschlußteil-Einheit ermöglichen.

In Kenntnis der Versuchsergebnisse und der vorsteherden Erläuterungen läßt sich das Verhalten der elastomeren Verschlußteile, die eine Beschichtungs-Dicke aus Poly-(paraxylylen) von 0,1 bis 0,5 Mikrometer aufweisen, etwa folgendermaßen erklären :
Beim Herstellen von Plastikbehältern, insbesondere aber beim Herstellen von Glasbehältern für pharmazeutische Präparate sind Unebenheiten und Unregelmäßigkeiten an der Oberfläche der Flaschenmündung nicht zu vermeiden. Diese können gut durch das elastische Verhalten von Gummistopfen od. dgl. Verschlußteile ausgeglichen werden, so daß auch über längere Zeit der in einer Medikamentenflasche enthaltene pharmazeutische Wirkstoff gegen Eindringen von Feuchtigkeit und Keimen sicher geschützt ist.

Die bezüglich der Anpassungsfähigkeit des Gummistopfens an die vorerwähnten Unregelmäßigkeiten der Flaschenmündungs-Oberfläche erwünschte Elastizität hat sich andererseits als ein Nachteil bei der Verarbeitung der Gummistopfen od. dgl. in Abfüll- und Verschließanlagen erwiesen. Mit zur Erfindung gehörende Versuche haben gezeigt, daß Gummistopfen od. dgl. elastomere Verschlußteile im Mikrobereich oberflächlich deformiert werden und sich gegenseitig aneinander oder auch auf ebenen Flächen von schienenartigen Gleiteinrichtungen od. dgl. Zuführvorrichtungen von Abfüll- und/oder Verschließanlagen festsaugen und dadurch dort gebremst werden können. Außerdem wird ein schnelles und sicheres Eindrücken der Gummistopfen od. dgl. in die Mündung entsprechender Medikamentenflaschen od. dgl., namentlich, wenn diese aus Glas bestehen, wegen der hohen Elastizität der Gummistopfen od. dgl. erschwert. Bekannte elastomere Verschlußteile, die eine Poly-(paraxylylen)-Beschichtung mit einer Schichtdicke von 0,5 Mikrometer bis 2,0 Mikrometer aufweisen, vermeiden wegen der durch die Poly-(paraxylylen)-Beschichtung erreichten glatteren und härteren Oberfläche die voerwähnten Deformationen im Mikrobereich weitgehend, wodurch auch das Gleiten der Gummistopfen od. dgl. in Zuführvorrichtungen von Abfüll- und Verschließanlagen erheblich verbessert wird. Es hat sich aber bei entsprechenden, vorerwähnten Versuchen gezeigt, daß auch im Bereich zwischen 0,1 bis 0,5 Mikrometer Beschichtungsdicke die Gefahr von Deformationen im Mikrobereich und die Gefahr von Störungen bei der Verarbeitung in Abfüll- und Verschließanlagen noch ausreichend vermieden werden kann, während diese sehr dünne Schichtdicken von 0,1 bis 0,5 Mikrometer bei elastomeren Verschlußteilen noch besser die herstellungsbedingten Unregelmäßigkeiten an der Mündung von Medikamentenflaschen, namentlich solchen aus Glas, besser ausgleichen und insbesondere den Gasaustausch zwischen dem Inneren der Medikamentenflasche und deren Umgebung merkbar vermindern können. Dies dürfte damit zusammenhängen, daß der Bereich der erfindungsgemäßen Dicke der Poly-(paraxylylen)-Beschichtung noch ausreicht, um den Reibungskoeffizienten und auch die beim Aufsetzen des elastomeren Verschlußteils auf die Medikamentenflaschen benötigte Eindrückkraft zum einen genügend kleingehalten werden kann, andererseits sich ein elastomeres Verschlußteil mit einer vergleichsweise dünnen Beschichtung noch besser an Unebenheiten von Flaschenmündungen anpassen kann.

Die Erfindung wird nachfolgend an einem Ausführungsbeispiel in Verbindung mit der Zeichnung erläutert. Es zeigt :
- Fig. 1: eine im Schnitt gehaltene Seitenansicht durch den oberen Teil einer Medikamentenflasche, in deren Flaschenmündung ein elastomeres Verschlußteil eingesetzt ist, und
- Fig. 2: einen stark vergrößerten Ausschnitt aus dem elastomeren Verschlußteil gemäß dem dortigen Ausschnitt A.

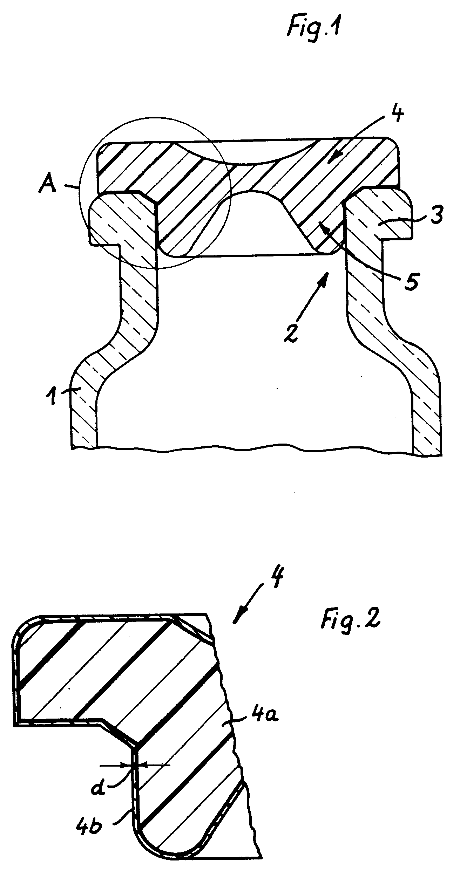

Fig. 1 zeigt den oberen Teil eines aus Glas bestehenden Behälters 1. Am äußeren Ende seiner Flaschenmündung 2 weist der Behälter 1 einen Befestigungsflansch 3 für ein elastomeres Verschlußteil 4 auf. Dieses ragt mit einem inneren Abschnitt 5 stopfenartig in die Flaschenmündung 2 hinein und verschließt somit den Behälter 1.

Fig. 2 zeigt einen vergrößerten Ausschnitt aus dem elastomeren Verschlußteil 4 gemäß dem Teilbereich A in Fig. 1. Dabei ist in Fig. 1 das elastomere Grundteil, das z. B. aus Gummi od. dgl. elastomerem Werkstoff besteht, mit 4a bezeichnet. Die dieses Grundteil 4a vollständig und durchgehend umgebende Schicht 4b aus Poly-(paraxylylen) weist erfindungsgemäß eine Dicke d von 0,1 bis 0,5 Mikrometer auf. Diese Schicht 4b ist in Fig. 2 der Deutlichkeit wegen verdickt und als gegenüber dem Grundteil 4a scharf abgegrenzte Schicht dargestellt.

## Patentansprüche

1. Verfahren zum Abfüllen und Verschließen von vorzugsweise aus Glas bestehenden Behältern mit pharmazeutisch reinem Inhalt, wobei die Behälter abgefüllt und anschließend mit einem elastomeren Verschlußteil verschlossen werden, der ein mit einer dünnen Beschichtung aus Poly-(paraxylylen) versehenes elastomeres Grundteil (Basisteil) aufweist, **dadurch gekennzeichnet,** daß das elastomere Grundteil (4a) vollständig und durchgehend mit einer Poly-(paraxylylen)- Beschichtung (4b) von einer Beschichtungsdicke (d) von 0,1 bis ausschließlich 0,5 Mikrometer (0,1 bis ausschließlich 0,5 microns) umhüllt wird.

2. Verfahren nach Anspruch 1, dadurch gekennzeichnet, daß die Beschichtungsdicke (d) der Poly-(paraxylylen)-Beschichtung (4b) derart gewählt wird, daß der Reibungskoeffizient des so beschichteten Verschlußteiles (4) kleiner als 1 ist.

3. Verfahren nach Anspruch 1 oder 2, dadurch gekennzeichnet, daß die Beschichtungsdicke (d) der Poly-(paraxylylen)-Beschichtung(4b) derart gewählt wird, daß die Vakuumretension des Verschlußteiles (4) während 24 h eine Erhöhung eines Vakuums von ursprünglich -846 mbar auf einen Wert größer als -300 mbar nicht zuläßt.

4. Verfahren nach wenigstens einem der Ansprüche 1 bis 3, dadurch gekennzeichnet, daß die Beschichtungsdicke (d) der Poly-(paraxylylen)-Beschichtung (4b) derart gewählt wird, daß der Gewichtsverlust einer Flasche, die zur Hälfte mit einer Lösung von 0,9 % Kochsalz und 0,4 % Phenol gefüllt ist, bei 16stündiger Lagerung im Hochvakuum von -1016 mbar nicht größer ist als 0,9 mg.

5. Verfahren nach wenigstens einem der Ansprüche 1 bis 4, dadurch gekennzeichnet, daß die Beschichtungsdicke (d) der Poly-(paraxylylen)-Beschichtung(4b) derart gewählt wird, daß der Austausch von Stickstoff gegen Luft nach 24 Stunden 2 % der ursprünglichen Stickstoffmenge nicht überschreitet.

6. Verfahren nach einem der Ansprüche 1 bis 5, dadurch gekennzeichnet, daß die Beschichtungsdicke (d) der Poly-(paraxylylen)-Beschichtung(4b) derart gewählt wird, daß die zum Aufsetzen auf die Medikamentenflaschen benötigte Eindrückkraft etwa 75 % bis 50 % unter der eines unbeschichteten elastomeren Verschlußteils liegt.

7. Verfahren nach einem der Ansprüche 1 bis 6, dadurch gekennzeichnet, daß das Verschlußteil (4) als gasdichtes Verschlußteil bei Medikamentenflaschen verwendet wird.

## Claims

1. A method of filling and closing containers, preferably glass containers, with pharmaceutically pure contents, the container being filled and then closed with an elastomeric closure piece having an elastomeric base part provided with a thin coating of poly(paraxylylene), **characterized in that** the elastomeric base part (4a) is completely and continuously covered by a poly(paraxylylene) coating (4b) of a coating thickness (d) of 0.1 to 0.5 micrometre inclusive (0.1 to 0.5 micron inclusive).

2. A method as claimed in claim 1, characterized in that the coating thickness (d) of the poly(paraxylylene) coating (4b) is selected in such a way that the coefficient of friction of the thus coated closure piece (4) is less than 1.

3. A method as claimed in claim 1 or claim 2, characterized in that the coating thickness (d) of the poly(paraxylylene) coating (4b) is selected in such a way that in the course of 24 hours the vacuum retention of the closure piece (4) does not allow a vacuum of originally -846 mbar to be increased to a value greater than -300 mbar.

4. A method as claimed in at least one of claims 1 to 3, characterized in that the coating thickness (d) of the poly(paraxylylene) coating (4b) is selected in such a way that the loss in weight of a bottle half filled with a solution of 0.9% common salt and 0.4% phenol during storage for 16 hours in high vacuum of -1016 mbar is no greater than 0.9 mg.

5. A method as claimed in at least one of claims 1 to 4, characterized in that the coating thickness (d) of the poly(paraxylylene) coating (4b) is selected in such a way that after 24 hours the exchange of nitrogen for air does not exceed 2% of the original amount of nitrogen.

6. A method as claimed in any one of claims 1 to 5, characterized in that the coating thickness (d) of the poly(paraxylylene) coating (4b) is selected in such a way that the press-in force needed for placement on the medicine bottles is about 75% to 50% under that of an uncoated elastomeric closure piece.

7. A method as claimed in any one of claims 1 to 6, characterized in that the closure piece (4) is used as a gasproof closure piece in medicine bottles.

## Revendications

1. Procédé pour emplir et fermer des récipients consistant avantageusement en du verre et contenant du produit pharmaceutique pur, selon lequel les récipients sont emplis puis fermés à l'aide d'une pièce élastomère de fermeture qui présente une partie de base en un élastomère comportant un mince revêtement en du poly(paraxylylène), procédé caractérisé en ce que la partie élastomère de base (4a) est entièrement et continûment enrobée d'un revètement en poly(paraxylylène) (4b) ayant une épaisseur (d) de revêtement de 0,1 à 0,5 micromètre exclu (0,1 à 0,5 micron exclu).

2. Procédé selon la revendication 1, caractérisé en ce que l'épaisseur (d) du revêtement (4b) en poly(paraxylylène) est choisie de façon que le coefficient de frottement de la pièce de fermeture (4) ainsi revêtue soit inférieur à 1.

3. Procédé selon la revendication 1 ou 2, caractérisé en ce que l'épaisseur (d) du revêtement (4b) en poly(paraxylylène) est choisie de façon que la rétention de vide par la pièce de fermeture (4) ne permet pas, en vingtquatre heures, à un vide valant initialement -846 mbars de s'élever à une valeur supérieure à -300 mbars.

4. Procédé selon l'une au moins des revendications 1 à 3, caractérisé en ce que l'épaisseur (d) du revêtement (4b) en poly(paraxylylène) est choisie de façon que la perte de poids d'une bouteille emplie à moitié d'une solution à 0,9 % de sel de cuisine et à 0,4 % de phénol ne soit pas supérieure à 0,9 mg lors d'un magasinage durant seize heures sous un vide poussé de -1016 mbars.

5. Procédé selon l'une au moins des revendications 1 à 4, caractérisé en ce que l'épaisseur (d) du revêtement (4b) en poly(paraxylylène) est choisie de façon que le remplacement de l'azote par de l'air au bout de vingt-quatre heures n'excède pas 2% de la quantité d'azote présente à l'origine.

6. Procédé selon l'une des revendications 1 à 5, caractérisé en ce que l'épaisseur (d) du revêtement (4b) en poly(paraxylylène) est choisie de façon que la force de pression nécessaire pour placer la pièce sur la bouteille de médicament se situe à environ 75 % à 50 % au-dessous de celle nécessaire pour une pièce de fermeture en de l'élastomère non revêtu.

7. Procédé selon l'une des revendications 1 à 6, caractérisé en ce que la pièce (4) de fermeture sert de pièce de fermeture étanche au gaz dans le cas de bouteilles ou flacons de médicament(s).
